# EUROPÄISCHE PATENTSCHRIFT

(11) **EP 1 942 914 B1**
(45) Veröffentlichungstag und Bekanntmachung des Hinweises auf die Patenterteilung: **20.01.2010**
(21) Anmeldenummer: 06828511.3
(22) Anmeldetag: 04.11.2006
(51) Int. Cl.: A61K 33/06, A61P 13/12, A61P 1/16, A61P 1/18, A61K 33/08

(54) **VERWENDUNG EINES AKTIVIERTEN ZEOLITHEN ALS PHARMAZEUTISCHES MITTEL FÜR DIE REDUZIERUNG GIFTIGER STOFFE**
USE OF AN ACTIVATED ZEOLITE AS A PHARMACEUTICAL AGENT FOR REDUCING POISONOUS SUBSTANCES
UTILISATION D'UNE ZEOLITE ACTIVEE COMME AGENT PHARMACEUTIQUE POUR REDUIRE DES SUBSTANCES TOXIQUES

(30) Priorität: 04.11.2005 DE 102005053090
(43) Veröffentlichungstag der Anmeldung: 16.07.2008
(73) Patentinhaber: Danz, Hubert, 38838 Eilenstedt (DE); Hoffmann, Steffen, 38838 Schlanstedt (DE); Görner, Thomas, 38838 Schlanstedt (DE); Woge, Oliver, 34474 Diemelstadt (DE)
(72) Erfinder: Danz, Hubert, 38838 Eilenstedt (DE); Hoffmann, Steffen, 38838 Schlanstedt (DE); Görner, Thomas, 38838 Schlanstedt (DE); Woge, Oliver, 34474 Diemelstadt (DE)
(74) Vertreter: Sperling, Thomas
(86) Internationale Anmeldenummer: PCT/DE2006/001951
(87) Internationale Veröffentlichungsnummer: WO 2007/051463

(56) Entgegenhaltungen:
- EP-A1- 1 316 530
- WO-A2-02/100420
- CERRI, G. ET AL: "Zeolites in biomedical application: Zn-exchanged clinoptilolite-rich rock as active carrier for antibiotics in anti-acne topical therapy" APPLIED CLAY SCIENCE , 27(3-4), 141-150 CODEN: ACLSER; ISSN: 0169-1317, 2004, XP008080410
- LI Y ET AL: "Ammoxidation of ethane - V. Solid-state ion exchange to prepare cobalt zeolite catalysts" APPLIED CATALYSIS A: GENERAL, ELSEVIER SCIENCE, AMSTERDAM, NL, Bd. 188, Nr. 1-2, 5. November 1999 (1999-11-05), Seiten 211-217, XP004271998 ISSN: 0926-860X

## Beschreibung

Die Erfindung betrifft die Verwendung eines aktivierten Zeolithen als pharmazeutisches Mittel für die Reduzierung unerwünschter giftige Stoffe im menschlichen oder tierischen Organismus gemäss der Ansprüche.

Schädliohe Umwelteinflüsse und Nahrungs- und Genussmittel belasten den Körper mit diversen Substanzen, die vom Körper nicht abgebaut werden können, sich über die Zeit im Organismus ansammeln und direkt oder mittelbar zu gesundheitlichen Störungen und zur Überlastung der Stoffwechselorgane führen können.

In ähnlicher Weise entstehen Belastungen durch Schwermetalle durch zahnmedizinische Behandlungen beispielsweise bei der Entfernung von Amalgamfüllungen oder Ähnlichem.

Das Adsorptionsvermögen von Zeolithen allgemein und für Schwermetallionen im Besonderen ist seit längerem in der Wissenschaft bekannt.

Nach der EP 1 316 530 B1 ist die Verwendung von 0,5 µm großen Zeolithen zur Verwendung als pharmazeutisches Mittel allgemein bekannt. Dabei werden Anwendungen zur Behandlung von Stoffwechsel-, sowie Herz-/Kreislauferkrankungen sowie rheumatischen Erkrankungen, multipler Sklerose sowie dermatologischen Erkrankungen beschrieben. Die pharmazeutischen Wirkungen treten nach der genannten Schrift durch den Zerkleinerungsgrad der Zeolithpartikel ein, welche durch ihre Größe im Verdauungstrakt des Menschen resorbierbar sind.
Damit ist auch ein Nachteil des genannten Standes der Technik verbunden, weil die Zeolithe teilweise vom Organismus aufgenommen werden und in diesem verbleiben. Des Weiteren besteht der Nachteil, dass häufig bei der angeblichen Entgiftung des menschlichen Organismus wichtige Stoffe wie z. B. Zink mit adsorbiert und unwirksam gemachte werden und so der Körper stoffliche Defizite bei Anwendung bzw. längerem Verzehr aufweisen könnte.

Die Aufgabe der Erfindung besteht darin, ein pharmazeutisches Mittel für die wirksame Entgiftung des menschlichen Körpers von Schwermetallen und Ammonium bereitzustellen.

Die Aufgabe der Erfindung wird durch die Verwendung eines in besonderer Weise zerkleinerten und aktivierten Zeoliths (MAC) gelöst, der bestimmte Oberflächeneigenschaften aufweist. Die erfindungsgemäße Substanz wird auch als modifizierter und aktivierter Zeolith bezeichnet.

Die Partikel des Zeolithen weisen dabei eine Partikelgröße von 6 bis 9 µm auf. Die Zetapotenziale des Zeolithen bewegen sich in einem Bereich von 24 bis 26 mV. Seine spezifische Oberfläche beträgt 35 bis 36 m²/g.

Die Konzeption der Erfindung besteht darin, dass der modifizierte und aktivierte Zeolith eine Partikelgröße und Oberflächeneigenschaften aufweist, welche eine optimale Adsorption der unerwünschten Bestandteile ermöglicht und dass anschließend die adsorptiv mit den unerwünschten Stoffen beladenen Zeolithe aus dem Organismus ausgetragen werden können. Letzteres wird vorteilhaft dadurch erreicht, dass die Partikel eine erfindungsgemäße Größe aufweisen und nicht vom Organismus resorbiert werden können.

Die Adsorptionsfähigkeit von Schwermetallen und die Bindung von Ammonium wird somit erfindungsgemäß zur Entlastung des Stoffwechsels eingesetzt.
Die erfindungsgemäße Verwendung des Zeolith ist sowohl im Verdauungstrakt des menschlichen oder tierischen Organismus als auch bei äußerlicher Anwendung gegeben.

Erfindungsgemäß wird dabei ein natürlich vorkommender Zeolith Klinoptilolith verwendet. Klinoptilolithe sind hydratisierte Alumosilicate (Zeolithe) mit einer Bruttoformel (Ca, K₂,Na₂, Mg)₄Al₈Si₄₀O₉₆ * 24 H₂O). Der erfindungsgemäße Klinoptilolith ist ein Calciumaluminiumsilikat.

Die natürlichen Vorkommen bestehen zu 84 % aus reinem Klinoptilolith. Die weitere mineralogische Zusammensetzung wird ergänzt durch:
- Cristobalit: 8 %;
- Feldspat: 3 bis 4 %;
- Illit: 4 %;
- Quarz: Spuren; und
- Carbonatminerale.

Chemisch ist das Mineral aus den folgenden Oxiden zusammengesetzt:
- SiO₂ 65 bis 71,3 %;
- Al₂O₃ 11,5 bis 13,1 %;
- CaO 2,7 bis 5,2 %;
- K₂O 2,2 bis 3,4 %;
- Fe₂O₃ 0,7 bis 1,9 %;
- MgO 0,6 bis1,2 %;
- Na₂O 0,2 bis 1,3 % und
- TiO₂ 0,1 bis 0,3 %.

Folgende physikalische Daten wurden ermittelt:

| | |
|---|---|
| Erweichungstemperatur : | 1260 °C; |
| Schmelztemperatur: | 1340 °C; |
| Dichte: | 2,2 bis 2,5 g/cm³; |
| Druckfestigkeit: | 33 Mpa; |
| Porösität: | 32 bis 40 %; |
| Porendurchmesser: | 0,4 nm und |
| Mohssche Härte: | 2,5 bis 3,5. |

Der Klinoptilolith ist grau-grün gefärbt und geruchlos.

Das Grundskelett des Klinoptilolith-Kristallgitters besteht aus SiO₂-Tetraedern und Al₂O₃. Das Verhältnis von Silizium zu Aluminium liegt beim Klinoptilolith bei 4:1. Die über Sauerstoffbrücken gebildeten Kristallstrukturen lassen Poren und Kanäle entstehen.

Durch den Austausch von vierwertigen Siliziumatomen durch dreiwertige Aluminiumatome entsteht eine Kationenaustauschkapazität. Dieser Austausch bewirkt einen negativen Ladungsüberschuss.

Die negativen Ladungen der Silizium/Aluminium-Tetraeder werden im Inneren durch positive Ionen der Alkali- und Erdalkalielemente (Ca, K, Na, Mg) sowie durch Wasser kompensiert.

Ein charakteristisches Merkmal der Zeolithe ist die selektive Austauschfähigkeit dieser gebundenen Ionen gegen andere (Selektivität: Cs⁺>Pb²⁺>NH₄⁺>Cu²⁺>Hg²⁺>Cd²⁺>Ni²⁺>Co²⁺, NH₄⁺>K⁺>Mg²⁺>Ca²⁺). Seine lonenaustauscherkapazität beträgt 1,2 bis 1,5 mol pro kg. Na⁺ und K⁺ sind dabei die dominantesten austauschbaren Kationen des Zeoliths.

Sie können durch andere Elemente ausgetauscht werden und wie in den folgenden Versuchen gezeigt wird, auch durch schädliche Elemente wie Blei, Ammonium: Quecksilber und andere Schwermetalle.

Die überraschende Wirkung des erfindungsgemäß bearbeiteten Klinoptilolithen besteht darin, das andererseits ein essenzielles Element wie Zink nicht oder nur unwesentlich adsorbiert wird.

Der Klinoptilolith ist in den verabreichten Mengen nicht giftig und wird, wie weitere Versuche zeigen, mit den durch den oben beschriebenen Prozess gebundenen Stoffen komplett aus dem menschlichen Organismus wieder ausgeschieden.

Der Klinoptilolith wurde erfindungsgemäß einer besonderen Bearbeitungsform unterzogen, wobei er zerkleinert, aktiviert und modelliert wurde.

Vor den Versuchen zur Wirksamkeit des Zeolithen wurden einige wichtige Partikeleigenschaften und Parameter, wie die spezifische Oberfläche, die Partikelgrößenverteilung und das ζ-Potenzial des aktivierten Klinoptiloliths, untersucht.

Die experimentelle Bestimmung der spezifischen Oberfläche wurde mit einem Messgerät Areameter II der Firma Juwe Laborgeräte GmbH durchgeführt, mit dem die Bestimmung der spezifischen Oberfläche nach dem von R. Haul und G. Dümbgen vereinfachten Messmethode nach DIN 66132 (Einpunkt-Methode) möglich ist. Das Messprinzip basiert auf der Tieftemperatur-Stickstoff-Adsorption. Dabei wurde die spezifische Oberfläche des erfindungsgemäßen Zeoliths mit 35,961 m²/g bestimmt.

Die Partikelgrößenverteilung wurde mit einem Messgerät Mastersizer 2000 der Firma Malvern mit Hilfe von Laserbeugung ermittelt. Die Messung der Partikelgröße erfolgte mit der Methode der Laserbeugung durch das Messgerät Mastersizer 2000 der Firma Malvem. Das erfindungsgemäße Klinoptilolithpulver wurde in einem Korngrößenbereich von 0,2 bis 20 µm untersucht. Dabei wurde der Hauptanteil der Partikel im Komgrößbereich von 6 bis 9 µm ermittelt.

Die ζ-Potenziale wurden mit dem Messgerät Zetamaster der Firma Malvern GmbH durchgeführt. Es wurden ζ-Potenfiale im Bereich von 24 bis 26 mV gefunden.

Ziel einer durchgeführten Studie war es also, die *in*-*vivo*-Systeme *in vitro* nachzustellen und die beschriebene Wechselwirkung von Schwermetallionen mit dem Zeolith Klinoptilolith quantitativ zu untersuchen. Als Modellsysteme wurden chemische Lösungen gewählt, die mit ihrer Zusammensetzung der Magensäure bzw. dem Zwölffingerdarm-Milieu nahe kommen. Die betrachteten Metallionen waren die von Quecksilber (Hg²⁺), Blei (Pb²⁺), Cadmium (Cd²⁺), Eisen (Fe³⁺), Zink (Zn²⁺), zusätzlich Ammonium (NH₄⁺).

Die Konzeption der Erfindung besteht bei oraler Applikation darin, dass die Partikel des Klinoptilolithen, mit den durch die Bearbeitung erreichten Partikelgrößen, nach Einnahme im menschlichen oder tierischen Verdauungstrakt wirken, ohne in die Zellen einzudringen. Das heißt, der aktivierte und modifizierte Klinoptilolith (MAC) wird im menschlichen oder tierischen Magen-Darm-Trakt nicht resorbiert und kann seine Wirkung überwiegend dort entfalten. Das Fehlen einer Resorption wurde durch Versuche mit Technetium-99 nachgewiesen. Mit dem Isotopenscanner konnte keine Strahlung in den Organen nachgewiesen werden, die durch eine Resorption von Klinoptilolith-Partikeln, die an das Technetium-99 fest verbunden waren, hervorgerufen worden wäre.

Im Folgenden werden die *in-vitro* durchgeführten Entgiftungsversuche beschrieben und die wichtigsten Ergebnisse aufgeführt.

Als synthetische Magensäure-Modelilösung diente ein Gemisch aus:
- 1,16 g Pepsin,
- 2,24 g KH₂PO₄,
- 0,69 g CaCl₂ * 2 H₂O,
- 0,42 g MgS0₄ * 7 H₂O und
- 4,00 g NaCl.

Dieses Gemisch wurde mit HCL auf 1 Liter aufgefüllt, wobei der pH-Wert der Lösung auf pH 1,5 eingestellt wurde.

Für die synthetische Zwölffingerdarmflüssigkeit wurde die oben genannte Zusammensetzung mit Ausnahme von KH₂PO₄ eingesetzt, da dies im alkalischen Milieu zu unkontrollierten Ausfällungen bzw. Co-Precipitationen der Schwermetallphosphate bzw. -hydroxide führen würde. Der pH-Wert wurde mit NaOH auf pH 8,1 eingestellt.

Zu der synthetischen Magensäure und der synthetischen Zwölffingerdarmlösung wurden die zu untersuchenden Schwermetalle bzw. Ammonium aus Standardlösungen zugegeben, so dass die folgende Zusammenstellung erreicht wurde:
- 10 mg/l Fe,
- 9 mg/l Zn,
- 0,9 mg/l Pb,
- 0,9 mg/l Cd,
- 0,9 mg/l Hg und
- 20 mg/l NH₄.

Von den so hergestellten Lösungen werden jeweils 100 ml mit Klinoptilolith ohne weitere Konditionierung zu 0 g/ 0,1 gl 0,2 g/ 0,3 g/ 0,4 g/ 0,5 g versetzt. Die Suspensionen wurden 90 min in einem PTFE-Gefäß geschüttelt. Nach Abschluss des Schüttelns wurden die Suspensionen zentrifugiert, die überstehende klare Flüssigkeit diente als Messlösung für die einzelnen Bestimmungen.

Die Bestimmung der Metallkonzentrationen erfolgte in den Lösungen mittels ICP-OES (Varian VISTA Pro) für Fe, Cd, Zn, Pb und mittels Kaltdampf-AAS (MWS DMA 80) für Hg.

Die Bestimmung des Ammoniums erfolgte mittels Photometrie bei 655 nm (Filterphotometer LP2W).

Zur Bestimmung der Säurebindungskapazität wurden 0,5 g Klinoptilolith mit 100 ml synthetischer Magensäure oben genannten Bedingungen behandelt und die freie Säure nach Abtrennung vom Klinoptilolithen mit 0,1 n NaOH-Lösung titriert. Analog wurde mit einer unbehandelten Magensäure verfahren.

Die Versuche zum Adsorptionsverhalten von Blei bei pH 1,5 zeigen, dass bei pH 1,5 eine ausgeprägte Adsorption von Blei stattfindet. Bei 0,5 g des eingesetzten Klinoptiloliths werden 32 % des eingesetzten Bleis absorbiert.

Ebenfalls eine sehr starke Adsorption von Blei findet bei entsprechenden Versuchen in der synthetischen Zwölffingerdarmlösung bei pH 8,1 statt. Bei 0,5 g des eingesetzten Klinoptiloliths werden 86 % des eingesetzten Bleis adsorbiert. Neben einer echten Adsorption könnte eine Mitfällung von Bleihydroxid mit den Eisenhydroxidkomplexen für eine sehr gute Abtrennung verantwortlich sein.

Die Versuche zum Adsorptionsverhalten von Quecksilber zeigen in der synthetischen Magensäure bei pH 1,5 eine gute Adsorption. Bei 0,5 g des eingesetzten Klinoptiloliths werden 57 % des eingesetzten Quecksilbers abgetrennt.

Die Versuche zum Adsorptionsverhalten von Quecksilber zeigen in der synthetischen Zwölffingerdarm-Modelllösung bei pH 8,1 eine gegenüber pH 1,5 verschlechterte Adsorption des Quecksilbers. Bei 0,5 g des eingesetzten Klinoptiloliths werden aber immerhin noch 45 % des Quecksilbers abgetrennt.

Neben den Schwermetallen wurde auch das Adsorptionsverhalten des erfindungsgemäßen Klinoptilolithen (MAC) gegenüber Ammoniumionen bestimmt. Die Versuche bei pH 1,5 zeigen keine von der angewendeten Klinoptilolithmenge abhängige Adsorption der Ammoniumionen.

Die Versuche bei pH 8,1 zeigen dagegen eine deutliche Adsorption der Ammoniumionen. Bei 0,5 g des eingesetzten Klinoptilolithen werden 36 % des Ammoniums abgetrennt. Eine Ursache könnte darin liegen, dass das Ammoniak/Ammonium-Gleichgewicht (NH₃/NH₄⁺) bei pH 8,1 mehr in Richtung Ammoniak (NH₃) verschoben wird und dieser sich an der Oberfläche des Klinoptilolithen anlagert.

Ein weiterer wichtiger Teil der Untersuchungen beschäftigte sich mit der Frage, ob essenzielle Elemente nicht auch adsorbiert werden, was bei längerer Einnahme zu gefährlichen Mangelerscheinungen führen würde.

Zunächst wurde dabei das Adsorptionsverhalten von Eisen in der synthetischen Magensäure-Modelllösung bei pH 1,5 bestimmt. Die Versuche zeigen, dass bei diesem pH-Wert Eisen aus dem Klinoptilolithen in die Messlösung abgegeben wird. Eine Zunahme der Eisenkonzentration in Abhängigkeit von der angewendeten Klinoptilolithmenge ist deutlich erkennbar. Bei 0,5 g des eingesetzten Klinoptilolithen beträgt die Zunahme der Eisenkonzentration 11 %, bezogen auf die Ausgangskonzentration.

Analog zu den vorhergehenden Versuchen wurde das Adsorptionsverhalten der Eisenionen auch in der synthetischen Zwölffingerdarmlösung mit dem pH 8,1 getestet. Diese Versuche zeigen, dass bei pH 8,1 die Eisenkonzentration in der Lösung abnimmt. Eine echte Adsorption von Fe²⁺ bzw. Fe³⁺ ist bei pH 8,1 unwahrscheinlich; eher dürfte eine Aggregation von Eisenhydroxykomplexen auf dem Klinoptilolithen stattfinden. Bei 0,5 g des eingesetzten Klinoptilolithen (MAC) beträgt die Abnahme des Eisens in der Lösung 14 %, bezogen auf die Ausgangskonzentration.

Das Verhalten gegenüber Zink als weiteres essenzielles Element wurde ebenfalls untersucht. Dabei zeigte sich überraschender Weise in der synthetischen Magensäure bei pH 1,5 erst bei größeren Mengen des Klinoptilolithen eine messbare Adsorption. Beim Einsatz von 0,5 g des Klinoptilolithen wurde etwa 1 % des Zinks absorbiert.

Ebenfalls gering ist die Adsorption von Zink bei gleichen Einsatzmengen von Klinoptilolith in der synthetischen Zwölffingerdarm-Lösung. Dort beträgt die Adsorption des eingesetzten Zinks bei 0,5 g Klinoptiloliths nur 1,6 %. Zur Bestimmung der Säurebindungskapazität wurde eine Lösung synthetische Magensäure mit 0,5 g Klinoptilolith geschüttelt und die freie Säure nach Abtrennung des Klinoptiloliths mit 0,1 n NaOH titriert. Zum Vergleich wurde eine unbehandelte Magensäure analysiert:
- unbehandelte Magensäure: 57,1 mmol HCl,
- mit 0,5 g Klinoptilolith behandelte Magensäure: 55,9 mmol HCl.

Zur Verifizierung dieses Ergebnisses wurde eine reine Salzsäure analog untersucht. Dabei enthielt die unbehandelte Lösung zunächst 93,7 mmol HCl. Die mit 0,5 g Klinoptilolith behandelte HCl-Lösung enthielt mit 91,8 mmol nur unwesentlich weniger Säure. Diese Ergebnisse lassen den Schluss zu, dass der Klinoptilolith keine nennenswerte Säurebindungskapazität aufweist.

Zusammenfassend lässt sich daher überraschender Weise feststellen: Die Untersuchungen bei pH 1,5 (nachgestelltes Magenmilieu) zeigen eine äußerordentlich gute Adsorptionsfähigkeit des Klinoptilolithen für Blei (32 %) und Quecksilber (57 %), während Zink (1 %) und Ammonium (1 %) unbeeinflusst bleiben. Für Eisen ist ein leichtes Ansteigen der Konzentration in der synthetischen Magensäure zu verzeichnen, dessen Ursache im Herauslösen von Eisen aus dem Klinoptilolithen zu suchen ist.

Bei pH 8,1 (nachgestellte Zwölffingerdarmflora) werden Blei (86 %) und Quecksilber (45 %) ebenfalls gut adsorbiert, während Zink keine Reaktion zeigt. Erstaunlich ist die gute Adsorption von Ammonium (36 %) in diesem Milieu. Geringe Anteile von Eisen können ebenfalls aufgenommen werden.
Es hat sich somit herausgestellt, dass dieser besonders bearbeitete erfindungsgemäße Klinoptilolith (MAC) wirklich enorme Fähigkeiten besitzt, Schwermetalle und Ammoniak zu binden und diese aus dem menschlichen Körper herauszutransportieren. Eine Adsorptionsfähigkeit für das essenzielle Element Zink ist nicht zu beobachten.

In ähnlicher Weise entstehen Belastungen durch Schwermetalle durch zahnmedizinische Behandlungen beispielsweise bei der Entfernung von Amalgamfüllungen oder Ähnlichem.

Im Folgenden wird unter einem aktivierten und modifizierten Zeolith ein solcher verstanden, dessen Partikel eine Partikelgröße von 2 bis 9 µm, Zetapotenziale von 20 bis 26 mV und eine spezifische Oberfläche von 30 bis 38 m²/g aufweisen.

Die Verwendung eines aktivierten und modifizierten Zeolithen als pharmazeutisches Mittel erfolgt für Erkrankungen der Stofiwechselorgane, insbesondere der Leber, der Nieren und der Bauchspeicheldrüse durch die Adsorption von Schwermetallen wie Blei und Quecksilber und/oder Ammonium im Verdauungstrakt von Menschen und Tieren und deren Ausleitung aus dem Organismus.

Eine Verwendung eines aktiven und modulierten Zeolithen als Medizinprodukt zur Aufnahme und Ausleitung von Quecksilber und Amalgam im Zahn- und Kieferknochenbereich des Menschen besteht darin, dass bei der Amalgamentfernung provisorische Füllungen mit dem erfindungsgemäßen Zeolith vorgenommen werden bzw. die Füllungen Bestandteile des Zeolithen aufweisen, wobei diese Füllungen nach vorübergehenden Verbleib im Zahn oder dem Kiefernknochenbereich mit den aufgenommenen und adsorbierten unerwünschten Stoffen entfernt werden. Auch dabei weisen die Partikel des Zeolithen eine Partikelgröße von 2 bis 9 µm, Zetapotenziale von 20 bis 26 mV und eine spezifische Oberfläche von 30 bis 38 m²/g auf.

## Patentansprüche

1. Verwendung eines aktivierten und modifizierten Zeoliths zur Herstellung eines Arzneimittels zur therapeutischen Behandlung von durch Absorption von Schwermetallen, insbesondere Blei und Quecksilber, und/oder Ammonium verursachten Erkrankungen der Stoffwechselorgane im Verdauungstrakt von Menschen, insbesondere der Leber, der Nieren und der Bauchspeicheldrüse, sowie zur Aufnahme und Ausleitung von Schwermetallen, wobei die Partikel des Zeoliths eine Partikelgröße von 2 bis 9 µm, Zetapotenziale von 20 bis 26 mV und eine spezifische Oberfläche von 30 bis 38 m²/g aufweisen.

2. Verwendung eines aktivierten und modifizierten Zeoliths nach Anspruch 1, **dadurch gekennzeichnet, dass** Quecksilber und Amalgam als Schwermetallkomponenten im Zahn- und Kiefernknochenbereich aufgenommen und ausgeleitet werden.

## Claims

1. Use of an activated and modified zeolite for the production of a medicament for the therapeutic treatment of diseases of the metabolic organs in the digestive tract of humans, in particular the liver, the kidneys and the pancreas, caused by the adsorption of heavy metals, in particular lead and mercury, and/or ammonia, and for the uptake and evacuation of heavy metals, wherein the particles of the zeolite have a particle size of 2 to 9 µm, zeta potentials of 20 to 26 mV and a specific surface of 30 to 38 m²/g.

2. Use of an activated and modified zeolite according to Claim 1, **characterized in that** mercury and amalgam as heavy metal components in the tooth and jawbone region are taken up and evacuated.

## Revendications

1. Utilisation d'une zéolithe activée et modifiée pour la fabrication d'un médicament destiné au traitement thérapeutique de maladies d'un organe métabolique dans le système digestif de l'homme, notamment du foie, des reins et du pancréas, provoquées par des métaux lourds, notamment par du plomb ou du mercure et/ou de l'ammonium, ainsi qu'à l'absorption et à l'évacuation de métaux lourds, les particules de la zéolithe présentant une dimension de particules de 2 à 9 µm, des potentiels zêta de 20 à 26 mV et une surface spécifique de 30 à 38m²/g.

2. Utilisation d'une zéolithe activée et modifiée selon la revendication 1, **caractérisée en ce que** le mercure et de l'amalgame sont absorbés et évacués sous forme de composantes de métaux lourds dans la région dentaire et maxillaire.
